# EUROPEAN PATENT APPLICATION

(11) **EP 2 266 461 A1**
(43) Date of publication of application: **29.12.2010**
(21) Application number: 10250988.2
(22) Date of filing: 27.05.2010
(51) Int. Cl.: A61B 5/1473, G01N 27/30

(54) **Flexible indwelling electrochemical-based biosensor and related methods**

(30) Priority: 28.05.2009 US 473953
(71) Applicant: Lifescan, Inc., Milpitas, CA 95035 (US)
(72) Inventor: Savage, Donna, Rolling Hills Estates, CA 90274 (US); Krulevitch, Peter, Pleasanton, CA 94566 (US); Zhao, Mingqi, San Jose, CA 95129 (US)
(74) Representative: Tunstall, Christopher Stephen

(57) **Abstract**

A flexible indwelling electrochemical-based biosensor (100;200) includes an elongated framework (102;202) and an integrated electrochemical-based biosensor (114;214). The elongated framework is formed of an electrically conductive flexible material (e.g., a Nitinol) with a body portion, a sharp head (106;206), a distal end and a proximal end. The integrated electrochemical-based biosensor (such as an electrochemical-based glucose sensor) is integrated with the elongated framework in that the biosensor has a sensing element that is disposed over the body portion or sharp head of the elongated framework and a portion of the elongated framework is configured as an electrode component that electrically cooperates with the sensing element. The electrode component can, for example, be configured to electrically cooperate as a working electrode, counter electrode, reference electrode or combined reference/counter electrode of the sensing element. Moreover, the sharp head is disposed at the distal end of the elongated framework and the sharp head, the electrode component and at least the sensing element of the biosensor are configured for insertion into a target site (for example, a subcutaneous target site).

## Description

### BACKGROUND OF THE INVENTION

Field of the Invention

The present invention relates, in general, to medical devices and, in particular, to indwelling biosensors and related methods.

Description of Related Art

A variety of indwelling biosensors are of interest to the scientific and medical community. For example, indwelling biosensors for continuous glucose monitoring have recently become available. These biosensors are subcutaneously inserted below a user's skin using a separate insertion device (e.g., a rigid hollow needle). The separate insertion device is removed after the biosensor is deployed. The biosensor then remains below the user's skin to continuously measure glucose concentrations in the user's interstitial fluid for an extended period of time.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1A is a simplified depiction of a portion of a flexible indwelling electrochemical-based biosensor according to an embodiment of the present invention;
FIG. 1B is a simplified cross-sectional depiction of the portion of a flexible indwelling electrochemical-based biosensor of FIG. 1A taken along line B-B of FIG. 1A;
FIG. 1C is a simplified cross-sectional depiction of the portion of a flexible indwelling electrochemical-based biosensor of FIG. 1A taken along the length of FIG. 1A;
FIG. 2 is a simplified perspective view of the distal end of a flexible indwelling electrochemical-based biosensor (with dashed lines indicating a channel and signal transmitting line of the biosensor that are hidden from view in the perspective of FIG. 2) according to an embodiment of the present invention;
FIG. 3A is a simplified depiction of a portion of a flexible indwelling electrochemical-based biosensor according to an embodiment of the present invention wherein an elongated framework is formed of an electrically conductive material and serves as a counter electrode;
FIG. 3B is a simplified diagram and electrical schematic depiction that includes the portion of a flexible indwelling electrochemical-based biosensor of FIG. 3A;
FIGs. 3C is a simplified cross-sectional depiction of the embodiment of FIG. 3B taken along line C-C of FIG. 3B;
FIG. 4A is a simplified depiction of a portion of a flexible indwelling electrochemical-based biosensor according to yet another embodiment of the present invention wherein a flexible elongated framework has a portion thereof configured as a working electrode base, and wherein a conductive layer is disposed on the working electrode base, the working electrode base and conductive layer constituting a working electrode of the electrochemical-based biosensor;
FIG. 4B is a simplified diagram and electrical schematic depicting the portion of a flexible indwelling electrochemical-based biosensor of FIG. 4A;
FIG. 4C is a simplified cross-sectional depiction of the embodiment of FIG. 4B taken along line C-C of FIG. 4B;
FIG. 5A is a simplified depiction of a portion of a flexible indwelling electrochemical-based biosensor according to another embodiment of the present invention wherein a flexible elongated framework has a portion thereof configured as a combined reference and counter electrode base and wherein a conductive layer is disposed on the combined reference and counter electrode base;
FIG. 5B is a simplified diagram and electrical schematic depicting the portion of a flexible indwelling electrochemical-based biosensor of FIG. 5A;
FIG. 5C is a simplified cross-sectional depiction of the embodiment of FIG. 5B;
FIG. 6 is a flow diagram depicting stages in a process for employing a flexible indwelling electrochemical-based biosensor according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

Flexible indwelling electrochemical-based biosensors according to embodiments of the present invention include an elongated framework and an integrated electrochemical-based biosensor. The elongated framework is formed, at least partially, from an electrically conductive flexible material (e.g., a Nitinol) with a body portion, a sharp head, a distal end and a proximal end. The integrated electrochemical-based biosensor (such as an electrochemical-based glucose sensor) is integrated with the elongated framework in that the biosensor has a sensing element that is disposed over at least one of the body portion or sharp head of the elongated framework and a portion of the elongated framework is configured as an electrode component that electrically cooperates with the sensing element. The electrode component can be, for example, configured to electrically cooperate (i.e., function) as a counter electrode, a working electrode base a reference electrode base or combined reference and counter electrode base of the sensing element.

Moreover, the sharp head is disposed at the distal end of the elongated framework and the sharp head, the electrode component and at least the sensing element of the biosensor are configured for insertion into a target site (for example, a subcutaneous target site). Further features, characteristics and benefits of such flexible indwelling biosensors are described below with respect to various drawings.

One skilled in the art will recognize that a biosensor is a device that detects and produces a signal related to a physiological change, process or analyte (such as information regarding glucose concentration in interstitial fluid). Such biosensors include those based on enzymatic reactions combined with electrochemical techniques (also referred to as electrochemical-based biosensors). Relevant, but non-limiting, examples of biosensors are described in U.S. Patent Nos. 7,498,132 B2, 7,429,630 B2, 7,109,271 B2, 7,465,380 B2 and 7,351,770 B2, each of which is hereby incorporated by reference as if fully set forth.

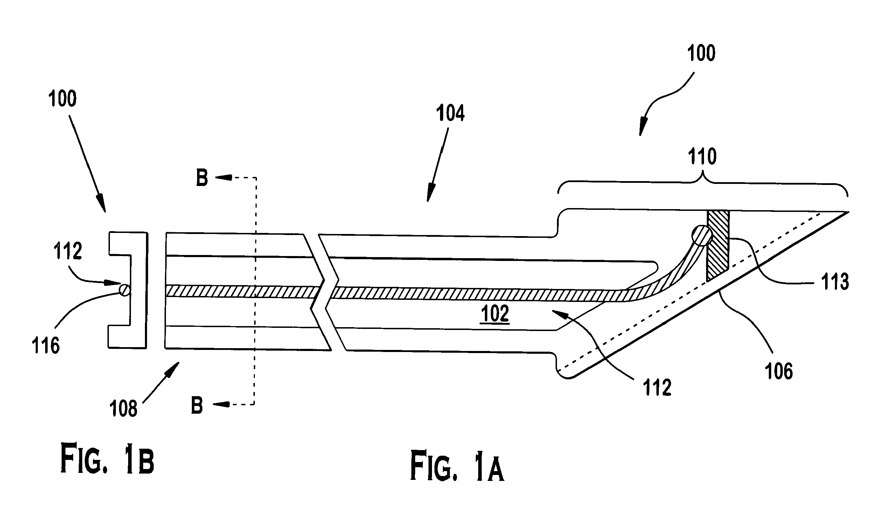

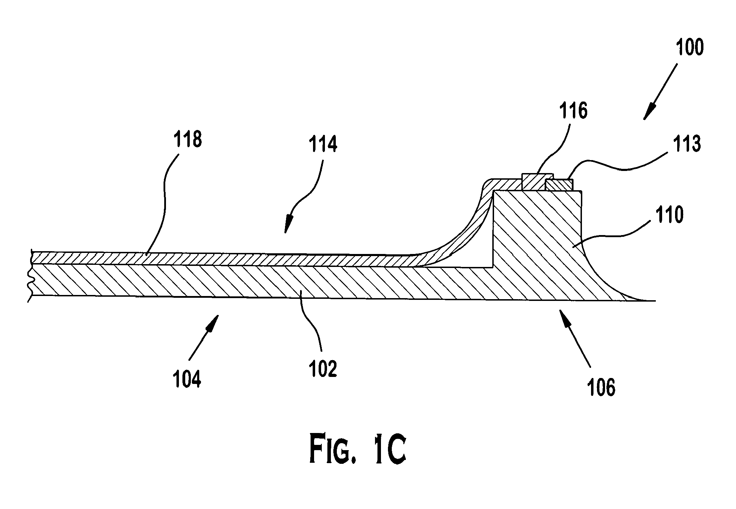

FIGs. 1A, 1B and 1C depict, in a simplified manner, portions of a flexible indwelling electrochemical-based biosensor 100 according to an embodiment of the present invention. Referring to FIGs. 1A through 1C, flexible indwelling electrochemical-based biosensor 100 includes an elongated strip 102 (i.e., an elongated framework) formed from a flexible electrically-conductive material (such as a Nitinol material, other suitable flexible or superelastic electrically conductive material) with a body portion 104, a distal end 106, a proximal end 108, a sharp head 110 disposed at distal end 106, a channel 112 and an electrode component 113.

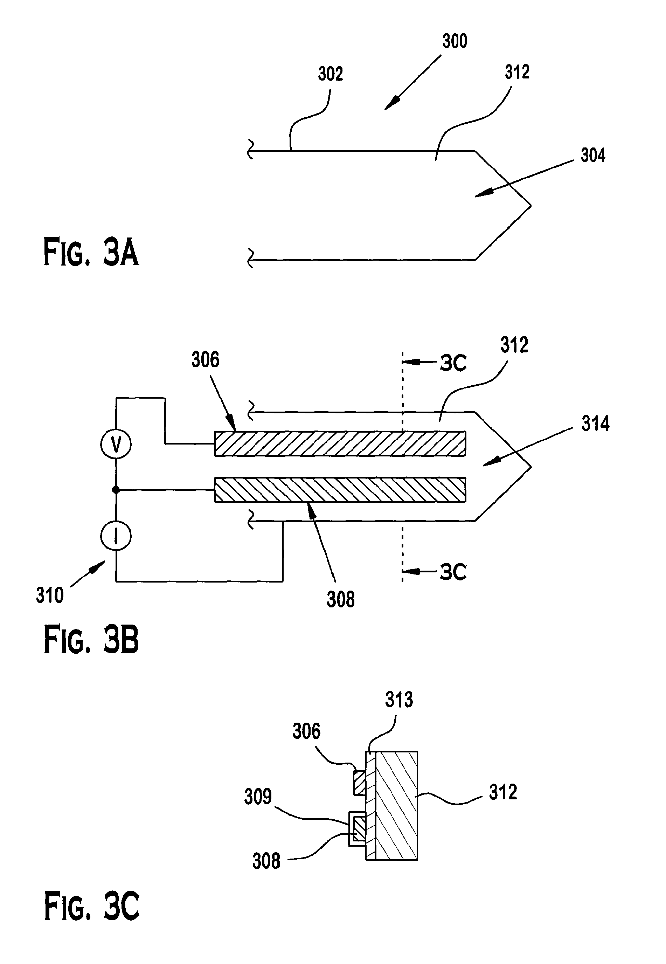

Channel 112 extends along the length of the elongated strip 102. Sharp head 110 is configured for subcutaneous skin insertion. FIG. 1A illustrates an embodiment in which a single edge of sharp head 110 is sharp (as indicated by the dashed line of FIG. 1A). Electrode component 113 is configured to electrically cooperate with sensing element 116 (described below) as an electrode (e.g., a counter electrode) or an electrode base (e.g., a working electrode base or a combined reference and counter electrode base). Such electrical cooperation is described further elsewhere herein with respect to the embodiments of FIGs. 3A through 7C.

Flexible indwelling electrochemical-based biosensor 100 also includes a biosensor 114 (shown in cross-hatching) that has a sensing element 116 and a signal transmitting line 118. Once apprised of the present invention, one skilled in the art will recognize that biosensor 114 can include a signal transmitting line of any suitable type including, for example, a co-axial cable, optical cable, a paired two-wire line, or a three-wire line. Moreover, biosensors employed in flexible indwelling electrochemical-based biosensors according to embodiments of the present invention can transmit signals using wireless methodologies including those that employ radio frequency (RF) and capacitive coupling techniques. In the embodiment of FIGs. 1A-1C, signal transmitting line 118 runs the length of the elongated strip 102 in channel 112.

Biosensor 114 is integrated with the elongated framework and sensing element 116 is securely positioned on sharp head 110 using, for example, a suitable adhesive (not shown). Moreover, sharp head 110, electrode component 113 and sensing element 116 are configured for insertion into a subcutaneous target site. The integration of biosensor 114 with the elongated framework includes cooperative electrical integration with electrode component 113.

Flexible indwelling electrochemical-based biosensors according to embodiments of the present invention are beneficial in that, for example, they can be consistently inserted to a predetermined depth below the skin, are comfortably flexible while being kink-resistant, and have a relatively small cross-sectional area. Moreover, since the flexible framework includes an electrode component that electrically cooperates with the sensing element, the flexible indwelling electrochemical-based biosensors are compact and relatively simple to manufacture.

Once apprised of the present disclosure, one skilled in the art will recognize that sensing elements employed in flexible indwelling biosensors according to embodiments of the present invention can be generally disposed on and/or suspended over the body portion or the sharp head of the elongated framework. FIGs. 1A-1C depict the sensing element on a sharp head. However, other configurations are possible including, for example, a configuration wherein the body portion of the elongated framework is cylindrical in shape with the transmission line of the biosensor being wound around the cylindrical elongated framework. Non-limiting alternative configurations that can be readily modified as embodiments of the present invention are described in co-pending U.S. Patent Application No. 12/366,466, which is hereby incorporated in full by reference.

Since flexible indwelling biosensors according to embodiments of the present invention can be formed with an elongated framework that is flexible and kink-resistant, they can have a relatively small cross-sectional area. It is hypothesized, without being bound, that such small cross-sectional areas result in minimal subcutaneous insertion pain and will be comfortable to wear.

Nitinol employed in embodiments of the present invention can be beneficially pre-processed (also referred to as preprogrammed) using techniques known to one skilled in the art to possess a variety of superelastic characteristics that are also known to those of skill in the art (such as, for example, kink-resistance, the ability to accommodate large loads and the ability to return to an original (preprogrammed) shape following release of mechanically deforming stresses).

Flexible indwelling electrochemical-based biosensor is very flexible, especially when bending such that the open side of the channels faces towards (or away from) the center of the radius of curvature, referred to as the flexible bending direction. Moreover, use of superelastic materials (such as Nitinol with a Young's modulus of in the range of approximately 35 to 75 GPa) provide for flexible indwelling electrochemical-based biosensors according to embodiments of the present invention to bend considerably without kinking.

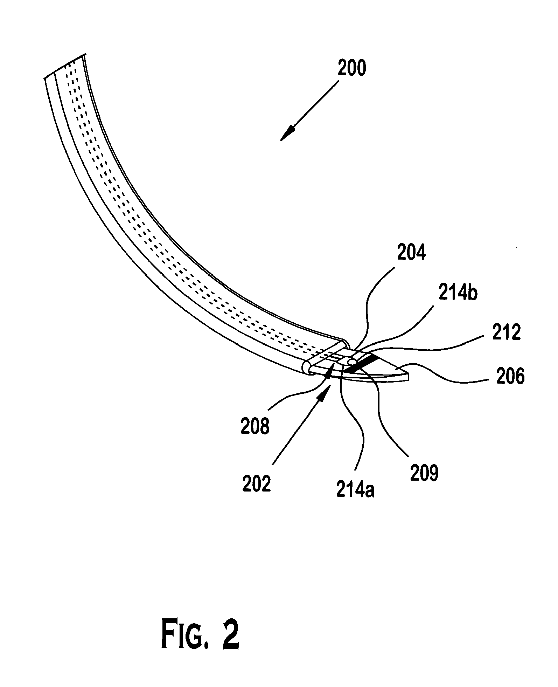

FIG. 2 is a simplified perspective view of the distal end of a flexible indwelling electrochemical-based biosensor 200 according to another embodiment of the present invention. Flexible indwelling electrochemical-based biosensor 200 includes a curved elongated framework 202 formed from a flexible material (such as Nitinol). Curved elongated framework 202 includes a body portion 204 and a sharp head 206. Curved elongated framework 202 also includes a channel 208 disposed along the longitudinal axis of curved elongated framework 202 and an electrode component 209. Electrode component 209 is configured to electrically cooperate with sensing element 212 of flexible indwelling electrochemical-based biosensor 200.

Flexible indwelling electrochemical-based biosensor 200 further includes a biosensor 210 that is integrated with curved elongated framework 202. Biosensor 210 includes a sensing element 212 disposed on sharp head 206 and two signal transmission lines 214a and 214b (depicted as a single dashed line within channel 208) that are partially contained within channel 208. Once apprised of the present disclosure, one skilled in the art will recognize that sensing elements employed in flexible indwelling biosensors according to the present invention can be also be disposed on the body portion of the elongated framework.

In the embodiment of FIG. 2, sharp head 206 is disposed at the distal end of curved elongated framework 202. Moreover, sharp head 206, electrode component 209 and sensing element 212 are configured for insertion into a target site.

Methods for manufacturing flexible frameworks suitable for use in flexible indwelling electrochemical-based biosensors according to embodiments of the present invention include etching a channel into an elongated Nitinol strip and forming a sharp head on a distal end of the elongated Nitinol strip. Alternatively, stamping and/or coining techniques can be employed to form the channel and sharp head of embodiments of the current invention. Moreover, conventional sharpening techniques, such as grinding, can also be used to form the sharp head. When the electrode component is an electrode base (e.g., a working electrode base or combined reference and counter electrode base) additional electrode-related layers (such as conductive and electrically insulating layers) required to form a suitable operative working electrode or combined reference and counter electrode) can be formed using any suitable layer creation technique including printing and deposition techniques. When such additional electrode-related layers are employed, they can equally be considered part of the elongated flexible framework or part of the sensing element due to the integrated nature thereof.

A flexible indwelling electrochemical-based biosensor according to embodiments of the present invention can be formed, for example, from an etched elongated Nitinol strip (with a sharp head) with a heat shrunk poly(tetrafluoroethylene) or PTFE polymer jacket serving as a flexible tube. Moreover, flexible indwelling electrochemical-based biosensors according to embodiments of the present invention can be used with insertion devices such as those described in U.S. Patent Application No. 12/366,466.

The sharp head of flexible indwelling electrochemical-based biosensors according to embodiments of the present invention remains in the target site during use of the flexible indwelling biosensor (for example, during the detection of glucose in interstitial fluid) and is only removed, for example, when the entire flexible indwelling biosensor is removed from the target site. Since the flexible indwelling biosensor is highly flexible (for example, being formed of Nitinol and, optionally, a flexible polymer tube), it can remain inserted without undue pain or discomfort during use.

FIGs. 3A, 3B, and 3C are various simplified depictions of portions of a flexible indwelling electrochemical-based biosensor 300 according to an embodiment of the present invention. Referring to FIGs. 3A, 3B and 3C, flexible indwelling electrochemical-based biosensor 300 includes an elongated framework 302 formed from a flexible electrically conductive material such as Nitinol. Elongated framework 302 has an essentially rectangular cross-sectional shape and includes a sharp head 304. FIG. 3C depicts the rectangular cross-sectional shape of elongated framework 302. Although, for the purpose of illustration only, the cross-sectional shape of elongated framework is depicted as rectangular in shape, the cross-sectional shape of elongated framework employed in embodiments of the present invention can be any suitable shape including, for example, a circular cross-sectional shape.

Flexible indwelling electrochemical-based biosensor 300 also includes a sensing element that includes a reference electrode 306, and working electrode 308 and various electrical components 310 depicted schematically in FIG. 3B. Nitinol is electrically conductive and a portion 312 of elongated framework 302 is configured as a counter electrode component that cooperates electrically with the sensing element of flexible indwelling electrochemical-based biosensor 300 as depicted in particular in FIGs. 3B and 3C.

A porous insulation layer 313 (see FIG. 3C and not depicted in FIGs. 3A and 3B) of the sensing element serves to electrically isolate working electrode 308 and reference electrode 306 from counter electrode component 310. Porous insulation layer 313 can be formed of any suitable material including, for example, parylene, polystytrene, polymethylmethacrylate polyacrylic, polyvinylpyrrolidone, silane, self-assembled monolayers, self-assembled multilayers, or other suitable organic and inorganic materials.

Moreover, an enzymatic reagent layer 309 of the sensing element (depicted in FIG. 3C only) is disposed on working electrode 308. Enzymatic reagent layer 309 includes, for example, an enzyme, a mediator, and either a binding agent or polymer matrix. A polymer matrix included in the enzymatic reagent layer can be formed of a polymeric material with a covalently bonded redox mediator and a hydrophilic affinity bonded enzyme. Such a redox polymer matrix can have, for example, a hydrophobic backbone and hydrophilic branches. The hydrophobic backbone can be formed of poly(methyl methacrylate) (PMMA), poly(butyl methacrylate), or polystyrene. In such a redox polymer, redox media can be covalently bonded to the hydrophilic branches. The redox media can be any suitable redox mediator including, for example, ferrocene, osmium, quinone, and methylene blue.

Reference electrode 306 can be formed of any suitable material including, for example, an Ag/AgCl material. Working electrode 308 can also be formed of any suitable material including, for example, gold, platinum, palladium and carbon ink.

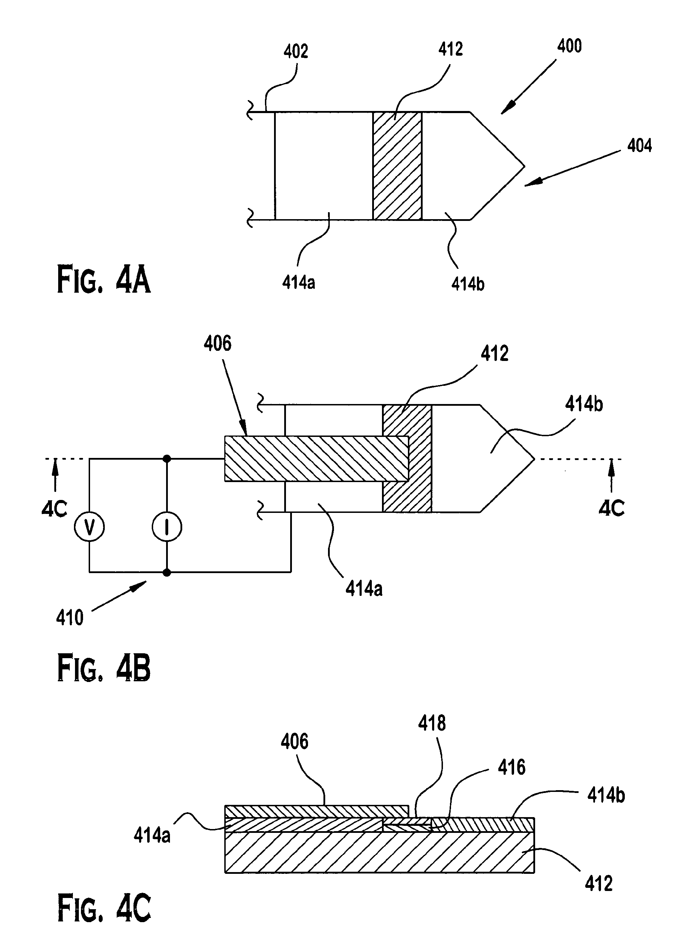

FIGs. 4A, 4B, and 4C are various simplified depictions of portions of a flexible indwelling electrochemical-based biosensor 400 according to an embodiment of the present invention. Referring to FIGs. 4A, 4B and 4C, flexible indwelling electrochemical-based biosensor 400 includes an elongated framework 402 formed from a flexible electrically conductive material such as Nitinol. Elongated framework 402 is essentially rectangular in cross-sectional shape and includes a sharp head 404.

Flexible indwelling electrochemical-based biosensor 400 also includes a sensing element that includes a combined reference/counter electrode 406, and various electrical components 410 depicted schematically in FIG. 4B. Nitinol is electrically conductive and a portion 412 of elongated framework 402 is configured as a working electrode base that cooperates electrically with the sensing element of flexible indwelling electrochemical-based biosensor 400 as depicted in particular in FIG. 4B. Elongated framework 402 also includes electrically insulating layers 414a and 414b that serve to electrically isolate the surface of portion 412 from the remaining surface of elongated framework 402. Combined reference/counter electrode 406 can be formed of any suitable reference electrode material including, for example, an Ag/AgCl material.

A conductive layer 416 (for example, an Au, Pt, Pd or carbon ink conductive layer) is disposed on working electrode base 412 to form a beneficially operative working electrode (as depicted in FIG. 4C only). Moreover, a combined enzymatic reagent and filter layer 418 (as depicted in FIG. 4C only) is disposed between combined reference and counter electrode 406 and the working electrode formed by the combination of conductive layer 416 and working electrode base 412.

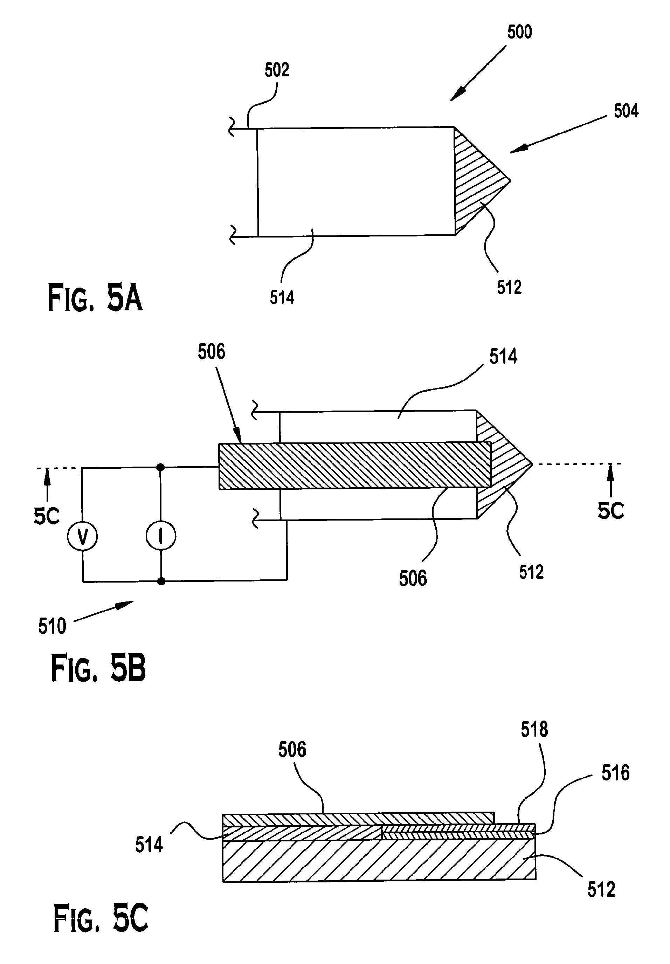

FIGs. 5A, 5B, and 5C are various simplified depictions of portions of a flexible indwelling electrochemical-based biosensor 500 according to an embodiment of the present invention. Referring to FIGs. 5A, 5B and 5C, flexible indwelling electrochemical-based biosensor 500 includes an elongated framework 502 formed from a flexible electrically conductive material such as Nitinol. Elongated framework 502 is essentially rectangular in cross-sectional shape and includes a sharp head 504.

Flexible indwelling electrochemical-based biosensor 500 also includes a sensing element that includes a working electrode 506, and various electrical components 510 depicted schematically in FIG. 5B. Nitinol is electrically conductive and a portion 512 of elongated framework 502 is configured as a combined reference/counter electrode base that cooperates electrically with the sensing element of flexible indwelling electrochemical-based biosensor 500 as depicted in particular in FIG. 5B. Elongated framework 502 also includes electrically insulating layer 514 that serves to electrically isolate the surface of portion 512 from the remaining surface of elongated framework 502.

Portion 512 is configured as a combined reference/counter electrode by including layer 516, formed of any suitable reference electrode material including, for example, an Ag/AgCl material, thereon (see FIG. 5C only). Working electrode 506 can also be formed of any suitable material including, for example, gold, platinum, palladium and carbon ink. An enzymatic reagent layer is disposed on the working electrode 506 (not shown in FIG. 5C). Filter layer 518 (see FIG. 5C only) is disposed between working electrode 506 and the combined reference/counter electrode formed by combined reference/counter electrode base 512 and layer 516.

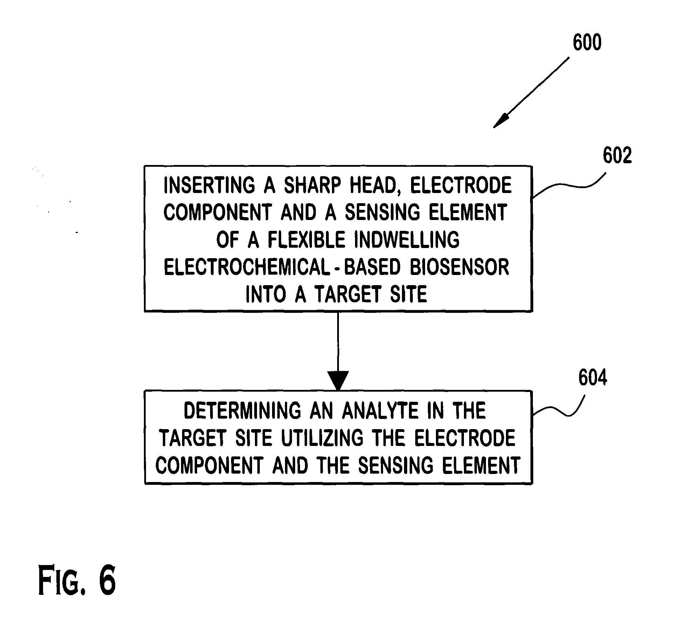

FIG. 6 is a flow diagram depicting stages in a method 600 for employing a flexible indwelling electrochemical-based biosensor according to an embodiment of the present invention. At step 602, method 600 includes inserting at least a sharp head, electrode component and a sensing element of a flexible indwelling electrochemical-based biosensor into a target site (such as a subcutaneous target site). Method 600 also includes determining an analyte (such as glucose) present in the target site by employing an electrochemical-based analytical technique that utilizes the electrode component and the sensing element of the flexible indwelling electrochemical-based biosensor, as set forth in step 604.

In method 600, the flexible indwelling electrochemical-based biosensor has an elongated framework, formed from an electrically conductive flexible material, and an integrated electrochemical-based biosensor with the integrated electrochemical-based biosensor includes the sensing element. Moreover, a portion of the elongated framework is configured as the electrode component (such as a working electrode base, counter electrode, reference electrode base or combined reference/counter electrode base) and the electrode component electrically cooperates with the sensing element during the determining step.

Once apprised of the present disclosure, one skilled in the art will recognize that method 600 can be readily modified to incorporate any of the procedures, uses, methodologies and actions described herein with respect to flexible indwelling electrochemical-based biosensors according to embodiments of the present invention.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

## Claims

1. A flexible indwelling electrochemical-based biosensor comprising:
an elongated framework formed from an electrically conductive flexible material, the elongated framework having:
a body portion;
a sharp head;
a distal end; and
a proximal end; and
an integrated electrochemical-based biosensor, the integrated electrochemical-based biosensor including:
a sensing element disposed over at least one of the body portion or sharp head of the elongated framework;
wherein the sharp head is disposed at the distal end of the elongated framework; and
wherein a portion of the elongated framework is configured as an electrode component that electrically cooperates with the sensing element; and
wherein the sharp head, the electrode component and at least the sensing element of the biosensor are configured for insertion into a target site.

2. The flexible indwelling electrochemical-based biosensor of claim 1 further including a flexible tube at least partially jacketing the elongated framework and the biosensor between the distal end and the proximal end of the elongated framework.

3. The flexible indwelling electrochemical-based biosensor of claim 1 wherein:
the elongated framework is an elongated strip with a longitudinal axis extending from the distal end to the proximal end; and
wherein the elongated strip has at least one channel formed therein, the at least one channel disposed at least partially parallel to the longitudinal axis; and
wherein the biosensor is at least partially contained within the at least one channel.

4. The flexible indwelling electrochemical-based biosensor of claim 1 wherein the electrode component is configured as a counter electrode of the sensing element.

5. The flexible indwelling electrochemical-based biosensor of claim 1 wherein the electrode component is configured as a working electrode base of the sensing element.

6. The flexible indwelling electrochemical-based biosensor of claim 5 further including a conductive layer disposed on the working electrode base.

7. The flexible indwelling electrochemical-based biosensor of claim 1 wherein the electrode component is configured as a combined reference and counter electrode base.

8. The flexible indwelling electrochemical-based biosensor of claim 7 further including a reference electrode material layer disposed on the combined reference and counter electrode base..

9. The flexible indwelling electrochemical-based biosensor of claim 1 wherein the sharp head is configured for subcutaneous insertion and the target site is a skin target site.

10. The flexible indwelling electrochemical-based biosensor of claim 1 wherein the electrically conductive flexible material is a superelastic electrically conductive flexible material.

11. The flexible indwelling electrochemical-based biosensor of claim 1 wherein the electrically conductive flexible material is Nitinol.

12. The flexible indwelling electrochemical-based biosensor of claim 1 wherein the sensing element is disposed on at least one of the sharp head and the body portion of the elongated framework.

13. A method for employing a flexible indwelling electrochemical-based biosensor, the method comprising:
inserting at least a sharp head, electrode component and a sensing element of a flexible indwelling electrochemical-based biosensor into a target site; and
determining an analyte present in the target site by employing an electrochemical-based analytical technique that utilizes the electrode component and the sensing element of the flexible indwelling electrochemical-based biosensor,
wherein the flexible indwelling electrochemical-based biosensor has an elongated framework, formed from an electrically conductive flexible material, and an integrated electrochemical-based biosensor, the integrated electrochemical-based biosensor including the sensing element; and
wherein a portion of the elongated framework is configured as the electrode component and the electrode component electrically cooperates with the sensing element during the determining step.
